# EUROPÄISCHE PATENTSCHRIFT

(11) **EP 0 834 505 B1**
(45) Veröffentlichungstag und Bekanntmachung des Hinweises auf die Patenterteilung: **07.01.2004**
(21) Anmeldenummer: 97116763.0
(22) Anmeldetag: 26.09.1997
(51) Int. Cl.: C07D 217/20, C07D 217/16, C07D 221/28

(54) **Verfahren zur Herstellung von (Z)-1-[1-(4-Methoxybenzyliden)-1,2,3,4,5,6,7,8-octahydro-isochinolin-2-yl]alkanonen**
Process for the preparation of (Z)-1-[1-(4-Methoxybenzylidene)-1,2,3,4,5,6,7,8-octahydro-isoquinolin-2-yl]alkanones
Procédé pour la préparation des (Z)-1-[1-(4-Méthoxybenzylidène)-1,2,3,4,5,6,7,8-octahydro-isoquinolin-2-yl]alkanones

(30) Priorität: 02.10.1996 EP 96115782
(43) Veröffentlichungstag der Anmeldung: 08.04.1998
(73) Patentinhaber: F. HOFFMANN-LA ROCHE AG, 4070 Basel (CH)
(72) Erfinder: Wehrli, Christof, 4108 Witterswil (CH)
(74) Vertreter: Kjellsaa-Berger, Hanny, Dr.

(56) Entgegenhaltungen:
- EP-A- 0 397 042
- DE-A- 2 311 881
- US-A- 4 857 648
- O. SCHNIDER ET AL.: HELVETICA CHIMICA ACTA, Bd. 33, Nr. 6, 1950, Seiten 1437-48, XP002050841
- M. WALTER ET AL.: HELVETICA CHIMICA ACTA, Bd. 44, Nr. 6, 1961, Seiten 1546-54, XP002050842
- M. KITAMURA ET AL.: TETRAHEDRON LETTERS, Bd. 28, Nr. 41, 1987, Seiten 4829-32, XP002050843
- M. KITAMURA ET AL.: JOURNAL OF ORGANIC CHEMISTRY, Bd. 59, Nr. 2, 1994, Seiten 297-310, XP002050844

## Beschreibung

Die vorliegende Erfindung betrifft ein neues Verfahren zur Herstellung von (Z)-1-[1-(4-Methoxybenzyliden)-1,2,3,4,5,6,7,8-octahydro-isochinolin-2-yl]-alkanonen der allgemeinen Formel 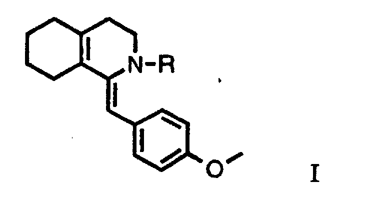 worin R C₂₋₇ - Alkanoyl bedeutet,
durch eine Bischler-Napieralski-Cyclisation von N-(2-Cyclohex-1-enylethyl)-2-(4-methoxyphenyl)acetamid der Formel 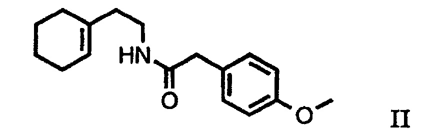 mit Phosphoroxychlorid und anschliessende Alkanoylierung des erhaltenen 1-(4-Methoxybenzyl)-3,4,5,6,7,8-hexahydro-isochinolins der Formel

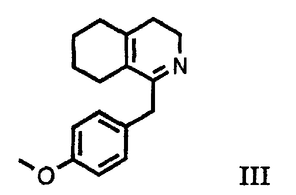

Der in dieser Beschreibung verwendete Ausdruck " C₂₋₇ - Alkanoyl" bedeutet sowohl geradkettige als auch verzweigte Reste einer aliphatischen Carbonsäure mit 2 bis 7 Kohlenstoffatomen, wie Acetyl, Propionyl, Butyryl, Valeryl, wobei Acetyl besonders bevorzugt ist.

Die (Z)-1-[1-(4-Methoxybenxyliden)-1,2,3,4,5,6,7,8-octahydro-isochinolin-2-yl]alkanone der obigen Formel I sind wichtige Ausgangsmaterialien zur Herstellung von Dextromethorphan der Formel

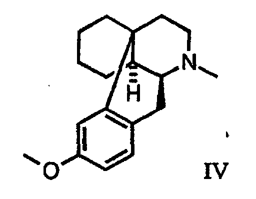

Sie können in an sich bekannter Weise, z.B. in Analogie zu den in der Deutschen Offenlegungsschrift 2 311 881 beschriebenen Methoden durch Reduktion der exocyclischen Doppelbindung, Cyclisation mit einer starken Säure zum entsprechenden Morphinanderivat, (Tetrahedron Letters, 1987, 28 (41), 4829-4832) Abspaltung des Restes R und N-Methylierung, in Dextromethorphan übergeführt werden. Die Verwendung der (Z)-1-[1-(4-Methoxybenzyliden)-1,2,3,4,5,6,7,8-octahydro-isochinolin-2-yl]alkanone zur Herstellung von Dextromethorphan ist ebenfalls Gegenstand der vorliegenden Erfindung.

Ein Verfahren zur Herstellung von 1-(4-Methoxybenzyl)-3,4,5,6,7,8-hexahydro-isochinolin aus N-(2-Cyclohex-1-enylethyl)-2-(4-methoxyphenyl)-acetamid ist in Helv. Chim. Acta 33, 1437 (1950) beschrieben. Nach diesem vorbekannten Verfahren wird das N-(2-Cyclohex-1-enylethyl)-2-(4-methoxyphenyl)acetamid der obigen Formel II mit einem Ueberschuss an Phosphoroxychlorid cyclisiert, und das erhaltene 1-(4-Methoxybenzyl)-3,4,5,6,7,8-hexahydro-isochinolin-hydrochlorid nach dem Basischstellen des Reaktionsgemisches in Form der freien Base mittels Extraktion isoliert. Allerdings ist die freie Base 1-(4-Methoxybenzyl)-3,4,5,6,7,8-hexahydro-isochinolin nicht lagerbeständig, weshalb es gemäss Helv. Chim. Acta 44, 1546 (1961) von Vorteil ist, nicht die freie Base, sondern das lagerbeständige Hydrochloridsalz mit Chloroform zu extrahieren. Grund für diese Chloroform-Extraktion ist die Tatsache, dass das erhaltene Isochinolinsalz praktisch nur mit einem chlorierten Lösungsmittel extrahiert werden kann. Da die Verwendung von einem Ueberschuss an Phosphoroxychlorid einerseits unökonomisch ist und andererseits die Verwendung von einem Ueberschuss dieses Reagens sowie von chlorierten Lösungsmitteln ohne aufwendige Entsorgung heute aus Gründen des Umweltschutzes nicht mehr akzeptiert wird, ist ein Verfahren unter Verwendung von möglichst geringen Mengen an Phosphoroxychlorid und ohne chlorierte Lösungsmittel von grossem technischem Interesse.

Die Herstellung von (Z)-1-[1-(4-Methoxybenzyliden)-1,2,3,4,5,6,7,8-octahydro-isochinolin-2-yl]ethanon aus isoliertem 1-(4-Methoxybenzyl)-3,4,5,6,7,8-hexahydro-isochinolin in einem mehrstufigen Verfahren ist auch in der US Patentschrift 4,857,648 beschrieben, doch ist die mit diesem Verfahren erzielbare Ausbeute für eine technische Anwendung ungenügend.

Der vorliegenden Erfindung liegt deshalb die Aufgabe zugrunde, ein Verfahren zur Herstellung von (Z)-1-[1-(4-Methoxybenzyliden)-1,2,3,4,5,6,7,8-octahydro-isochinolin-2-yl]alkanonen der obigen Formel I durch Cyclisation von N-(2-Cyclohex-1-enylethyl)-2-(4-methoxyphenyl)acetamid und anschliessende N-Alkanoylierung bereitzustellen, welches die Nachteile der vorbekannten Arbeitsweisen (Einsatz von überschüssigem Phosphoroxychlorid, mehrstufige Synthese, geringe Ausbeute und Verwendung eines chlorierten Lösungsmittels) nicht aufweist.

Im Rahmen der vorliegenden Erfindung wurde diese Aufgabe dadurch gelöst, dass die beiden Reaktionsschritte ohne Isolieren der Zwischenprodukte in einem Eintopfverfahren unter den weiter unten im Detail beschriebenen ganz spezifischen Reaktionsbedingungen durchgeführt werden.

Demnach betrifft die vorliegende Erfindung ein Verfahren zur Herstellung von (Z)-1-[1-(4-Methoxybenzyliden)-1,2,3,4,5,6,7,8-octahydro-isochinolin-2-yl]alkanonen der obigen Formel I durch eine Bischler-Napieralski-Cyclisation von N-(2-Cyclohex-1-enylethyl)-2-(4-methoxyphenyl)acetamid der obigen Formel II und anschliessende Alkanoylierung des erhaltenen 1-(4-Methoxybenzyl)-3,4,5,6,7,8-hexahydro-isochinolins der obigen Formel III unter einer Inertgasatmosphäre, welches dadurch gekennzeichnet ist, dass die Bischler-Napieralski-Cyclisation in Gegenwart von 0.45 bis 0.8 Moläquivalenten Phosphoroxychlorid erfolgt, die Reaktionslösung aus der Bischler-Napieralski-Cyclisation gegebenenfalls mit einem unter den Reaktionsbedingungen inerten aprotischen organischen Lösungsmittel verdünnt und das erhaltene 1-(4-Methoxybenzyl)-3,4,5,6,7,8-hexahydro-isochinolin *in situ*, d.h. ohne Isolieren, in Gegenwart einer unter den Reaktionsbedingungen inerten schwachen organischen Base alkanoyliert wird.

Die Bischler-Napieralski-Cyclisation erfolgt in an sich bekannter Weise in einem unter den Reaktionsbedingungen inerten aprotischen organischen Lösungsmittel bei einer Temperatur zwischen 70°C und einer Temperatur, welche unmittelbar unterhalb der Rückflusstemperatur des Reaktionsgemisches liegt. Für den Zweck der vorliegenden Erfindung haben sich aromatische Kohlenwasserstoffe, wie Toluol oder Xylol, Nitrile, wie Propionitril oder Butyronitril, insbesondere Toluol oder Propionitril, vor allem Toluol, als besonders geeignet erwiesen. Die Reaktionstemperatur liegt vorzugsweise zwischen 80° und 110°C, besonders bevorzugt zwischen 80° und 100°C. Bei Temperaturen unterhalb von 70°C wird die Bischler-Napieralski-Cyclisation zu langsam und oberhalb von 110°C entstehen vermehrt Nebenprodukte. Mit zunehmender Verdünnung entstehen zwar etwas weniger Nebenprodukte, die Ausbeute wird dadurch jedoch nicht verbessert. Es werden vorzugsweise 0.5 bis 0.55 Moläquivalent Phosphoroxychlorid eingesetzt. Die Zugaberate von Phosphoroxychlorid ist bei gleichbleibender Reaktionstemperatur nicht kritisch, obwohl eine langsame Zugabe die Ausbeute geringfügig verbessert. In einer besonders bevorzugten Ausführungsform wird das Phosphoroxychlorid bei 80°C kontinuierlich zugegeben und nach beendeter Zugabe das Reaktionsgemisch schrittweise oder kontinuierlich auf 100°C erwärmt.

Der Reaktionsschritt der Alkanoylierung wird ebenfalls in an sich bekannter Weise bei einer Temperatur zwischen Raumtemperatur und 80°C in Gegenwart von 2-4 Moläquivalenten (bezogen auf Phosphoroxychlorid) einer unter den Reaktionsbedingungen inerten schwachen organischen Base unter einer Inertgasatmosphäre, vorzugsweise unter Stickstoff oder Argon, durchgeführt, da sich die Gegenwart von auch nur Spuren von Sauerstoff sehr negativ auf die Ausbeute auswirkt. Um eine möglichst hohe Ausbeute zu erzielen, ist somit ein Sauerstoffausschluss absolut notwendig. Es hat sich deshalb als vorteilhaft erwiesen, die Alkanoylierung nicht nur unter einer Inertgasatmosphäre durchzuführen, sondern alle verwendeten Lösungsmittel und Reagentien sauerstofffrei einzusetzen. Obwohl der Sauerstoffausschluss nur für die Alkanoylierung zwingend ist, wird aus Gründen der Zweckmässigkeit bereits die Cyclisation mit Phosphoroxychlorid unter Sauerstoffausschluss durchgeführt.

Als Alkanoylierungsmittel können die Anhydride und Chloride, vorzugsweise die Anhydride, von C₂₋₇ - Alkancarbonsäuren verwendet. werden, besonders bevorzugt Acetanhydrid. Die Menge an eingesetztem Alkanoylierungsmittel soll 1-4 , vorzugsweise 2-3, Moläquivalente betragen, damit die Umsetzung mit dem 1-(4-Methoxybenzyl)-3,4,5,6,7,8-hexahydroisochinolin der obigen Formel III möglichst quantitativ verläuft.

Als unter den Reaktionsbedingungen inerte schwache organische Basen, welche sich für den Zweck der vorliegenden Erfindung eignen, kommen tert. Amine in Frage, vorzugsweise Dialkylaniline, z.B. Dimethylanilin oder Diethylanilin, Pyridin, Picoline, z.B. α-Picolin, Chinolin, Isochinolin. Die Verwendung von Dimethylanilin ist besonders bevorzugt.

Für den Reaktionsschritt der Alkanoylierung wird gegebenenfalls die Reaktionslösung aus der Bischler-Napieralski-Cyclisation mit einem unter den Reaktionsbedingungen inerten aprotischen organischen Lösungsmittel verdünnt. Für diesen Zweck geeignete Lösungsmittel sind insbesondere Ether, wie Methyl-t-butylether, Tetrahydrofuran und Dioxan, aromatische Kohlenwasserstoffe, wie Toluol, Nitrile, wie Acetonitril oder Propionitril. Bevorzugt wird mit dem gleichen Lösungsmittel, in welchem die Bischler-Napieralski-Cyclisation durchgeführt wurde, verdünnt. Besonders bevorzugt ist auch hier die Verwendung von Toluol.

Das folgende Beispiel zur Herstellung von (Z)-1-[1-(4-Methoxybenzyliden)-1,2,3,4,5,6,7,8-octahydro-isochinolin-2-yl]ethanon durch Cyclisation von N-(2-Cyclohex-1-enylethyl)-2-(4-methoxyphenyl)acetamid und anschliessende N-Alkanoylierung zeigt eine besonders vorteilhafte Ausführungsform des erfindungsgemässen Verfahrens auf, soll jedoch keinerlei Einschänkung darstellen. Alle Temperaturen sind in Celsiusgraden angegeben.

### Beispiel

In einem 350 ml Vierhalskolben mit Magnetrührer, Thermometer, Gasabzug mit Paraffinöl-Sperre und einer Motorkolbenbürette wurden 27.34 g (100 mMol) N-(2-Cyclohex-1-enylethyl)-2-(4-methoxyphenyl)acetamid in 30 ml Toluol vorgelegt. Der darin gelöste Sauerstoff wurde dadurch entfernt, dass man unter Rühren die Reaktionssuspension bis zum Sieden evakuierte und danach das Vakuum durch Zugabe von Argon aufhob (dieser Vorgang wird nachstehend als Inertisieren bezeichnet). Unter Rühren wurden bei 80° innerhalb von 60 Minuten 4.73 ml ( 51 mMol) destilliertes Phosphoroxychlorid zudosiert. Die Lösung liess man 1 Stunde bei 80°, danach 2 Stunden bei 90° und schliesslich nochmals 1 Stunde bei 100° nachreagieren. Das Reaktionsgemisch wurde danach mit 90 ml inertisiertem Toluol und dann mit 20.7 ml (220 mMol) inertisiertem Acetanhydrid versetzt. Dann wurden 19.01 ml (150 mMol) unter Inertgas destilliertes N,N-Dimethylanilin zugegeben. Die Reaktionsmischung wurde zunächst 18 Stunden bei 70° und danach 24 Stunden bei Raumtemperatur gerührt. Nach dem Abkühlen auf etwa 10° wurden 50 ml eiskaltes Wasser zugegeben. Nach 10 Minuten Rühren wurde die Toluolphase abgetrennt, und die wässrige Phase mit 2x100 ml Toluol extrahiert. Die 3 Toluolphasen wurden mit 3x 25 ml 1N eiskalter Salzsäure und 2x 25 ml Wasser gewaschen. Die Toluolphasen wurden anschliessend mit 1x 50 ml 2 molarer, eiskalter Natronlauge und 2x 25 ml Wasser gewaschen, vereinigt und im Wasserstrahlvakuum auf 52.2 g eingeengt (die Lösung enthielt das rohes (Z)-1-[1-(4-Methoxybenzyliden)-1,2,3,4,5,6,7,8-octahydroisochinolin-2-yl]ethanon sowie Toluol).

In einem 250 ml Dreihalsrundkolben mit Rückflusskühler, Thermometer und Plattenrührer wurden unter Inertgasatmosphäre die obigen 52.2 g Rückstand und 2.6 g eines Zweitkristallisates von (Z)-1-[1-(4-Methoxybenzyliden)-1,2,3,4,5,6,7,8-octahydro-isochinolin-2-yl]ethanon aus einem vorangegangenen Ansatz vorgelegt. Durch Erwärmen auf 60° wurde die Suspension gelöst. Dann wurden 27.5 g n-Hexan dazugegeben und die Lösung danach bei 50° mit (Z)-1-[1-(4-Methoxybenzyliden)-1,2,3,4,5,6,7,8-otahydro-isochinolin-2-yl]ethanon angeimpft. Die erhaltene Suspension wurde dann im Wasserbad unter Rühren auf 0° abgekühlt. Nach 18 Stunden bei 0° wurde abgenutscht und mit 10 ml eines eiskalten 4:6-Gemisches von Toluol und n-Hexan nachgewaschen. Das Kristallisat wurde bei 45° im Wasserstrahlvakuum bis zur Gewichtskonstanz getrocknet, wobei man 26.1 g eines Erstkristallisates von (Z)-1-[1-(4-Methoxybenzyliden)-1,2,3,4,5,6,7,8-octahydroisochinolin-2-yl]ethanon vom Schmelzpunkt 103-4° erhielt, Ausbeute 87%, Gehalt = 99% (nach HPLC).

Das Filtrat aus der Erstkristallisation wurde im Wasserstrahlvakuum eingedampft, wobei 5.5 g eines Rückstandes erhalten wurden. Dieser wasserfreie Rückstand wurde in 10 ml Toluol gelöst, und die Lösung inertisiert. Zu dieser Lösung wurden 1 ml einer 1-molaren Lösung von Acetylchlorid in Toluol und 0.5 ml einer 1-molaren Lösung von Essigsäure in Toluol gegeben. Die so erhaltene Lösung wurde zunächst 6 Stunden bei etwa 40° und danach etwa 18 Stunden bei Raumtemperatur unter einer Inertgasatmosphäre gerührt. Dann wurde bei Raumtemperatur in einer Portion eine Lösung von 12 ml einer 0.75-molaren Natronlauge in 50%igem wässrigem Methanol zugegeben. Nach 10-minütigem Rühren des Reaktionsgemisches bei Raumtemperatur wurde das zweiphasige Gemisch mit 2x 20 ml Toluol extrahiert. Die organischen Phasen wurden mit 2x 20 ml Wasser, 2x 20 ml 0.1 N Salzsäure und 2x 20 ml Wasser gewaschen, vereinigt und anschliessend im Wasserstrahlvakuum eingedampft. Der Rückstand wurde aus 20 g Diisopropylether kristallisiert. Nach 18 Stunden Stehenlassen bei 0° wurde das Kristallisat abgenutscht und mit 5 ml eiskaltem Diisopropylether nachgewaschen. Der Nutschkuchen wurde 4 Stunden bei 45° im Wasserstrahlvakuum getrocknet, wobei man 3.2 g eines Zweitkristallisates von (Z)-1-[1-(4-Methoxybenzyliden)-1,2,3,4,5,6,7,8-octahydro-isochinolin-2-yl]ethanon erhielt.

## Patentansprüche

1. Verfahren zur Herstellung von (Z)-1-[1-(4-Methoxybenzyliden)-1,2,3,-4,5,6,7,8-octahydro-isochinolin-2-yl)alkanonen der allgemeinen Formel 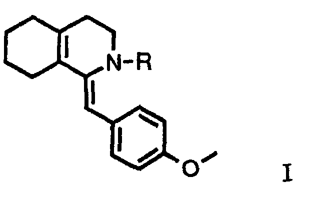 worin R C₂₋₇ - Alkanoyl bedeutet,
durch eine Bischler-Napieralski-Cyclisation von N-(2-Cyclohex-1-enylethyl)-2-(4-methoxyphenyl)acetamid der Formel 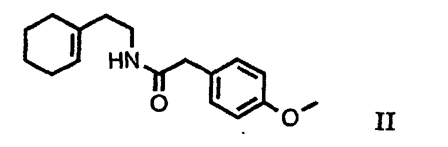 und anschliessende Alkanoylierung des erhaltenen 1-(4-Methoxybenzyl)-3,4,-5,6,7,8-hexahydro-isochinolins der Formel 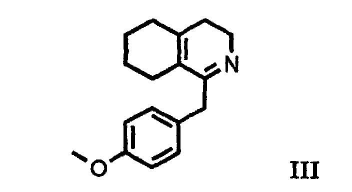 unter einer Inertgasatmosphäre, **dadurch gekennzeichnet, dass** die Bischler-Napieralski-Cyclisation in Gegenwart von 0.45 bis 0.8, vorzugsweise 0.5 bis 0.55, Moläquivalenten Phosphoroxychlorid und vorzugsweise ebenfalls unter einer Inertgasatmosphäre erfolgt und das erhaltene 1-(4-Methoxybenzyl)-3,4,5,6,7,8-hexahydro-isochinolin *in situ*, d.h. ohne Isolieren, in Gegenwart einer unter den Reaktionsbedingungen inerten schwachen organischen Base alkanoyliert wird.

2. Verfahren gemäss Anspruch 1, **dadurch gekennzeichnet, dass** für die Bischler-Napieralski-Cyclisation als ein unter den Reaktionsbedingungen inertes aprotisches organisches Lösungsmittel Toluol, Xylol, Propionitril oder Butyronitril, vorzugsweise Toluol oder Propionitril, besonders bevorzugt Toluol, verwendet wird und die Reaktionstemperatur zwischen 70°C und einer Temperatur liegt, welche unmittelbar unterhalb der Rückflusstemperatur des Reaktionsgemisches liegt, vorzugsweise zwischen 80° und 110°C, besonders bevorzugt zwischen 80° und 100°C, wobei bevorzugt das Phosphoroxychlorid bei 80°C kontinuierlich zugegeben und nach beendeter Zugabe das Reaktionsgemisch auf 100°C erwärmt wird.

3. Verfahren gemäss Anspruch 1 oder 2, **dadurch gekennzeichnet, dass** bei der Alkanoylierung als eine unter den Reaktionsbedingungen inerte schwache organische Base ein tert. Amin, vorzugsweise ein Dialkylanilin, Pyridin, ein Picolin, Chinolin oder Isochinolin, besonders bevorzugt Dimethylanilin, verwendet wird.

4. Verfahren gemäss einem der Ansprüche 1-3, **dadurch gekennzeichnet, dass** die Reaktionslösung aus der Bischler-Napieralski-Cyclisation mit einem unter den Reaktionsbedingungen inerten aprotischen organischen Lösungsmittel verdünnt wird.

5. Verfahren gemäss einem der Ansprüche 1-4, **dadurch gekennzeichnet, dass** bei der Alkanoylierung als zusätzliches Lösungsmittel das gleiche Lösungsmittel verwendet wird, in welchem die Bischler-Napieralski-Cyclisation durchgeführt wurde, und dass es sich hierbei um einen Ether, vorzugsweise Methyl-t-butylether, Tetrahydrofuran oder Dioxan, einen aromatischen Kohlenwasserstoff, vorzugsweise Toluol, ein Nitril, vorzugsweise Acetonitril oder Propionitril, besonders bevorzugt Toluol, handelt.

6. Verfahren gemäss einem der Ansprüche 1-5, **dadurch gekennzeichnet, dass** bei der Alkanoylierung 2-4 Moläquivalente (bezogen auf Phosphoroxychlorid) einer unter den Reaktionsbedingungen inerten schwachen organischen Base verwendet werden.

7. Verfahren gemäss einem der Ansprüche 1-6, **dadurch gekennzeichnet, dass** für die Alkanoylierung sauerstofffreie Lösungsmittel und Reagentien verwendet werden.

8. Verfahren gemäss einem der Ansprüche 1-7, **dadurch gekennzeichnet, dass** die Alkanoylierung bei einer Temperatur zwischen Raumtemperatur und 80°C und mit 1-4, vorzugsweise 2-3, Moläquivalenten Alkanoylierungsmittel durchgeführt wird.

9. Verfahren gemäss einem der Ansprüche 1-8, **dadurch gekennzeichnet, dass** als Alkanoylierungsmittel Acetanhydrid verwendet wird.

10. Verfahren zur Herstellung von Dextromethorphan der Formel 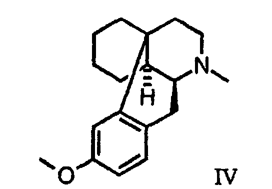 **dadurch gekennzeichnet, dass** man N-(2-Cyclohex-1-enylethyl)-2-(4-methoxyphenyl)acetamid der Formel 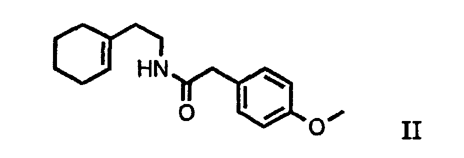 in Gegenwart von 0.45 bis 0.8 Moläquivalenten Phosphoroxychlorid cyclisiert, das erhaltene 1-(4-Methoxybenzyl)-3,4,5,6,7,8-hexahydro-isochinolin der Formel 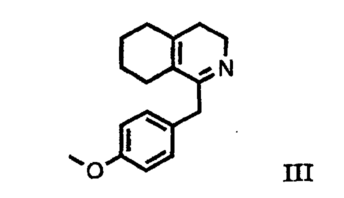 *in situ*, d.h. ohne Isolieren, in Gegenwart einer unter den Reaktionsbedingungen inerten schwachen organischen Base unter einer Inertgasatmosphäre alkanoyliert und ein erhaltenes (Z)-1-[1-(4-Methoxybenzyliden)-1,2,3,4,5,6,7,8-octahydro-isochinolin-2-yl]alkanon der allgemeinen Formel 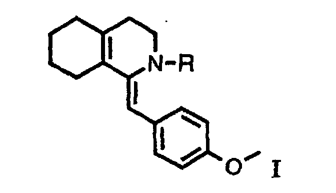 worin R C₂₋₇ - Alkanoyl bedeutet,
in an sich bekannter Weise durch Reduktion der exocyclischen Doppelbindung, Cyclisation mit einer starken Säure zum entsprechenden Morphinanderivat, Abspaltung des Restes R und N-Methylierung in Dextromethorphan überführt.

## Claims

1. A process for the production of (Z)-1-[1-(4-methoxybenzylidene)-1,2,3,4,5,6,7,8-octahydro-isoquinolin-2-yl]alkanones of the general formula 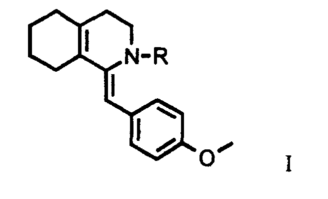 wherein R signifies C₂₋₇-alkanoyl,
by a Bischler-Napieralski cyclization of N-(2-cyclohex-1-enylethyl)-2-(4-methoxyphenyl)-acetamide of the formula 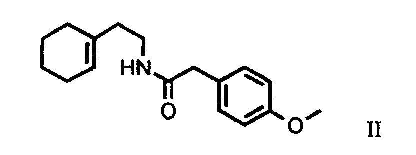 and subsequent alkanoylation of the resulting 1-(4-methoxybenzyl)-3,4,5,6,7,8-hexahydro-isoquinoline of the formula 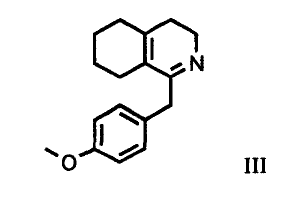 under an inert gas atmosphere, **characterized by** carrying out the Bischler-Napieralski cyclization in the presence of 0.45 to 0.8, preferably 0.5 to 0.55, molar equivalents of phosphorus oxychloride and preferably also under an inert gas atmosphere and alkanoylating the resulting 1-(4-methoxybenzyl)-3,4,5,6,7,8-hexahydro-isoquinoline *in situ*, i.e. without isolation, in the presence of a weak organic base which is inert under the reaction conditions.

2. A process according to claim 1, **characterized in that** toluene, xylene, propionitrile or butyronitrile, preferably toluene or propionitrile, especially toluene, is used as an aprotic organic solvent which is inert under the reaction conditions for the Bischler-Napieralski cyclization and the reaction temperature lies between 70°C and a temperature which lies just below the reflux temperature of the reaction mixture, preferably between 80° and 110°C, especially between 80° and 100°C, with the phosphorus oxychloride preferably being added continuously at 80°C and, after completion of the addition, the reaction mixture being heated to 100°C.

3. A process according to claim 1 or 2, **characterized in that** a tert. amine, preferably a dialkylaniline, pyridine, a picoline, quinoline or isoquinoline, especially dimethylaniline, is used in the alkanoylation as a weak organic base which is inert under the reaction conditions.

4. A process according to any one of claims 1-3, **characterized in that** the reaction solution from the Bischler-Napieralski cyclization is diluted with an aprotic organic solvent which is inert under the reaction conditions.

5. A process according to any one of claims 1-4, **characterized in that** the same solvent in which the Bischler-Napieralski cyclization has been carried out is used in the alkanoylation as the additional solvent and is an ether, preferably methyl t-butyl ether, tetrahydrofuran or dioxan, an aromatic hydrocarbon, preferably toluene, a nitrile, preferably acetonitrile or propionitrile, especially toluene.

6. A process according to any one of claims 1-5, **characterized in that** 2-4 molar equivalents (based on phosphorus oxychloride) of a weak organic base which is inert under the reaction conditions is used in the alkanoylation.

7. A process according to any one of claims 1-6, **characterized in that** oxygen-free solvents and reagents are used for the alkanoylation.

8. A process according to any one of claims 1-7, **characterized in that** the alkanoylation is carried out at a temperature between about room temperature and 80°C and using 1-4, preferably 2-3, molar equivalents of alkanoylating agent.

9. A process according to any one of claims 1-8, **characterized in that** acetic anhydride is used as the alkanoylating agent.

10. A process for the manufacture of dextromethorphan of the formula 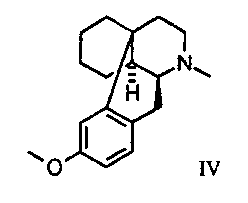 **characterized by** cyclizing N-(2-cyclohex-1-enylethyl)-2-(4-methoxyphenyt)acetamide of the formula 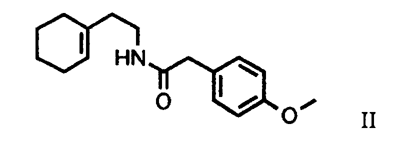 in the presence of 0.45 to 0.8 molar equivalents of phosphorus oxychloride, alkanoylating the resulting 1-(4-methoxybenzyl)-3,4,5,6,7,8-hexahydro-isoquinoline of the formula 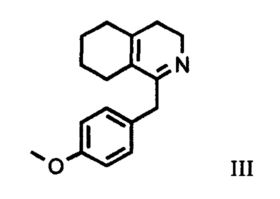 *in situ*, i.e. without isolation, in the presence of a weak organic base which is inert under the reaction conditions under an inert gas atmosphere and converting a resulting (Z)-1-[1-(4-methoxybenzylidene)-1,2,3,4,5,6,7,8-octahydro-isoquinolin-2-yl]alkanone of the general formula 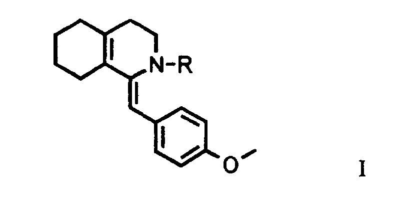 wherein R signifies C₂₋₇-alkanoyl,
into dextromethorphan in a manner known per se by reduction of the exocyclic double bond, cyclization with a strong acid to the corresponding morphinan derivative, cleavage of the residue R and N-methylation.

## Revendications

1. Procédé de préparation de (Z)-1-[1-(4-méthoxybenzylidène)-1,2,3,4,5,6,7,8-octahydro-isoquinoléin-2-yl]alcanones de formule générale 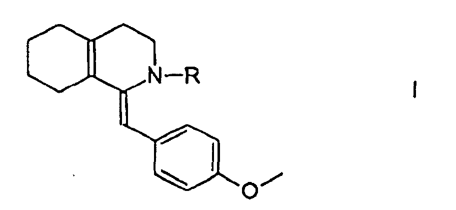 dans laquelle R correspond à un radical alcanoyle en C₂₋₇,
utilisant une cyclisation de Bischler et Napieralski du N-(2-cyclohex-1-enyléthyl)-2-(4-méthoxyphényl)acétamide de formule 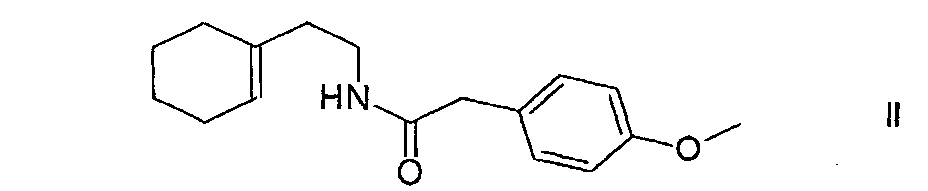 et une réaction d'alcanoylation du 1-(4-méthoxybenzyl)-3,4,5,6,7,8-hexahydro-isoquinoléine obtenue de formule 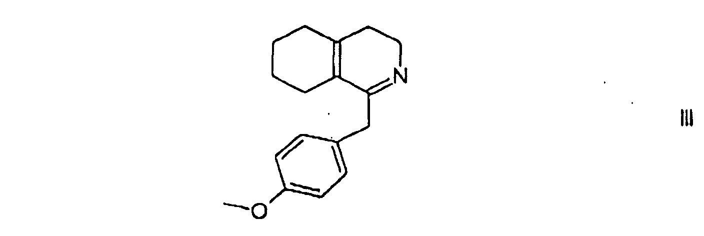 sous une atmosphère de gaz inerte, **caractérisé en ce que** la cyclisation de Bischler et Napieralski est effectuée en présence de 0,45 à 0,8 équivalents molaires, de préférence, de 0,5 à 0,55 équivalents molaires d'oxychlorure de phosphore, de préférence sous une atmosphère de gaz inerte, et l'alcanoylation de la 1-(4-méthoxybenzyl)-3,4,5,6,7,8-hexahydro-isoquinoléine obtenue est effectuée in situ, c'est à dire sans isolement, en présence d'une base organique inerte et faible dans les conditions de la réaction.

2. Procédé selon la revendication 1, **caractérisé en ce que** pour la cyclisation de Bischler et Napieralski, on utilise comme solvant organique inerte aprotique dans les conditions de la réaction le toluène, le xylène, le propionitrile ou le butyronitrile, de préférence le toluène ou le propionitrile, et plus préférentiellement le toluène la température de réaction est comprise entre 70°C et une température, qui se trouve directement au-dessous de la température de reflux du milieu réactionnel, laquelle est comprise, de préférence, entre 80° et 110°C, et encore plus préférentiellement entre 80° et 100°C ; de préférence, on ajoute l'oxychlorure de phosphore de façon continue à 80°C et après cette addition le milieu réactionnel est réchauffé à 100°C.

3. Procédé selon les revendications 1 ou 2, **caractérisé en ce que** pour l'alcanoylation comme base organique inerte et faible, sous les conditions de la réaction, est une amine tertiaire, de préférence une dialkylaniline, une pyridine, une picoline, une quinoléine ou une isoquinoléine, plus préférentiellement la diméthylaniline.

4. Procédé selon l'une des revendications 1 à 3, **caractérisé en ce que** la solution résultant de la cyclisation de Bischler et Napieralski est diluée, avec un solvant organique, aprotique qui sous les conditions de la réaction, est inerte.

5. Procédé selon une des revendications 1 à 4, **caractérisé en ce que** pour effectuer l'alcanoylation on utilise comme solvant supplémentaire le même solvant utilisé dans la cyclisation de Bischler et Napieralski, et ce solvant est un éther, de préférence, l'éther méthyl t-butyle, le tétrahydrofuranne ou le dioxanne, un hydrocarbure aromatique, de préférence, le toluène ou un nitrile, de préférence, l'acétonitrile ou le propionitrile, et plus préférentiellement, le toluène.

6. Procédé selon une des revendications 1 à 5, **caractérisé en ce que** de 2 à 4 équivalents molaires (par rapport à l'oxychlorure de phosphore) d'une base organique inerte et faible sous les conditions de la réaction sont utilisés pour effectuer l'alcanoylation.

7. Procédé selon une des revendications 1 à 6, **caractérisé en ce que** des solvants et des réactifs sans oxygène sont utilisés pour l'alcanoylation.

8. Procédé selon une des revendications 1 à 7, **caractérisé en ce que** l'alcanoylation est effectuée à une température comprise entre la température ambiante et 80°C et avec de 1 à 4 équivalents molaires, de préférence, de 2 à 3 équivalents molaires d'agent d'alcanoylation.

9. Procédé selon une des revendications 1 à 8, **caractérisé en ce que** l'anhydride acétique est utilisé comme agent d'alcanoylation.

10. Procédé de préparation de dextromethorphane de la formule IV 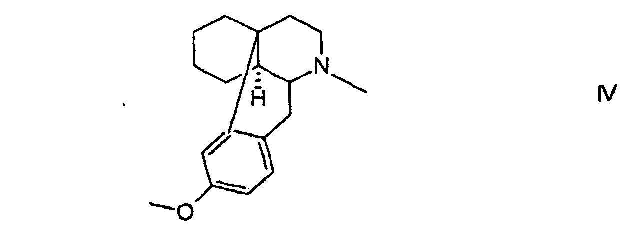 **caractérisé en ce que** le N-(2-cyclohex-1-enylethyl)-2-(4-méthoxy phényl)acétamide de formule 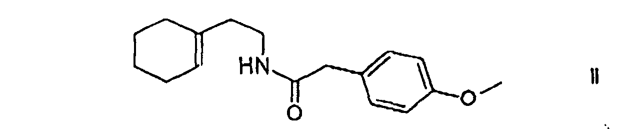 est cyclisé en présence de 0,45 à 0,8 équivalents molaires de chlorure de phosphoryle, le produit de cette réaction c'est à dire le 1-(4-méthoxybenzyl)-3,4,5,6,7,8-hexahydro-isoquinoléine de formule 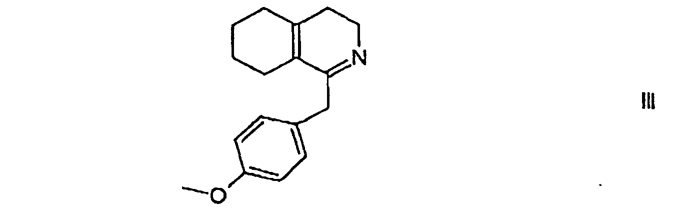 est alcanoylé in situ, c'est à dire sans isolement, en présence d'une base organique inerte et faible sous les conditions de la réaction sous une atmosphère de gaz inerte et le produit obtenu par cette réaction, la (Z)-1-[1-(4-méthoxybenzylidène)-1,2,3,4,5,6,7,8-octahydro-isoquinoléin-2-yl]alcanone de formule générale 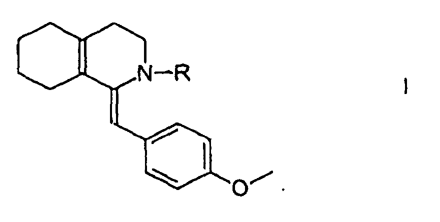 dans laquelle R correspond à un radical alcanoyle en C₂₋₇,
est converti en dextrométhorphane, de façon bien connue, en utilisant les réactions de réduction des doubles liaisons exocycliques, de cyclisation avec un acide fort d'élimination du reste R et de la méthylation de N.
